# FASCICULE DE BREVET EUROPEEN

(11) **EP 3 796 886 B1**
(45) Date de publication et mention de la délivrance du brevet: **12.04.2023**
(21) Numéro de dépôt: 19725731.4
(22) Date de dépôt: 23.05.2019
(51) Int. Cl.: A61K 8/34, A61K 36/898, A61Q 19/08, A61K 36/487, A61K 8/9783

(54) **EXTRAIT DE VANILLE DE TAHITI ET 4-[(1E,3S)-3-ÉTHENYL-3,7-DIMÉTHYLOCTA-1,6-DIENYL]PHÉNOL POUR LUTTER CONTRE LE VIEILLISSEMENT CUTANÉ**
TAHITI VANILLE-EXTRAKT UND 4-[(1E,3S)-3-ETHENYL-3,7-DIMETHYLOCTA-1,6-DIENYL]PHENOL ZUR BEKÄMPFUNG GEGEN HAUTALTERUNG
TAHITIAN VANILLA EXTRACT AND 4-[(1E,3S)-3-ETHENYL-3,7-DIMETHYLOCTA-1,6-DIENYL]PHENOL TO COMBAT SKIN AGEING

(30) Priorité: 23.05.2018 FR 1854294
(43) Date de publication de la demande: 31.03.2021
(73) Titulaire: Pierre Fabre Dermo-Cosmétique, 92100 Boulogne-Billancourt (FR)
(72) Inventeur: DUPLAN, Hélène, 31320 AUZEVILLE TOLOSAN (FR); BACQUEVILLE, Daniel, 31100 TOULOUSE (FR); POIGNY, Stéphane, 31600 SAUBENS (FR)
(74) Mandataire: Regimbeau
(86) Numéro de dépôt international: PCT/EP2019/063321
(87) Numéro de publication internationale: WO 2019/224303

(56) Documents cités:
- WO-A1-2008/140673
- FR-A1- 2 837 384
- FR-A1- 2 891 148
- DATABASE GNPD [Online] MINTEL; août 2011 (2011-08), "Complex", XP002784844, Database accession no. 2029779
- Ratan Chaudhuri: "Bakuchiol: A Retinol-Like Functional Compound, Modulating Multiple Retinol and Non-Retinol Targets" In: "Cosmeceuticals and Active Cosmetics, Third Edition", 27 août 2015 (2015-08-27), CRC Press, XP055507398, ISBN: 978-1-4822-1417-8 pages 1-18, DOI: 10.1201/b18895-2, le document en entier

## Description

### DOMAINE TECHNIQUE

La présente invention concerne l'utilisation d'une association de 4-[(1E,3S)-3-éthenyl-3,7-diméthylocta-1,6-dienyl]phénol et d'un extrait de vanille de Tahiti pour lutter contre le vieillissement de la peau, et les compositions cosmétiques ou dermatologiques contenant cette association destinée à lutter contre le vieillissement cutané.

### ETAT DE LA TECHNIQUE

La peau est l'organe du corps humain le plus étendu, près de 2m² de surface. La peau doit sa souplesse et sa résistance aux différentes couches de tissus qui la composent : l'épiderme, le derme et l'hypoderme. L'épiderme, couche extérieure de la peau, est responsable de son imperméabilité et de sa résistance. Il est essentiellement composé de kératine, une protéine fibreuse produite par les kératinocytes, et de mélanine, le principal pigment cutané fabriqué par les mélanocytes. Avec les années, le renouvellement des kératinocytes s'effectue plus lentement et leur différentiation terminale est ralentie. Au cours du temps, de profondes modifications se produisent au niveau du derme. Tissu de soutien de la peau, il est constitué à 80% de fibres d'élastine et de collagène noyées dans un gel de glycoprotéines. Les fibroblastes qui sont les principales cellules du derme, sont spécialisées dans la synthèse de ces fibres d'élastine et de collagène. Ils assurent l'équilibre entre synthèse, maturation et dégradation des fibres d'élastine et de collagène. Entre 20 et 80 ans, la population de fibroblastes diminue de moitié. Ainsi, dans le temps, cet équilibre va se déplacer vers une dégradation de ces fibres avec pour résultats, une perte d'élasticité et de tonicité du derme et une flaccidité qui ne s'oppose plus aux effets de contraction des muscles sous-jacents, conduisant à l'apparition de rides. Les fibres de collagène sont réparties dans toutes les couches du derme, elles sont capables de fixer l'eau et contribuent à l'hydratation de la peau. Une diminution du collagène et/ou une modification de sa qualité conduisent à l'apparition de rides profondes. Avec le vieillissement, les fibres d'élastine, qui confèrent élasticité et solidité aux tissus, se raréfient, la peau devient plus mince et se ride. La peau doit continuellement faire face à de multiples agressions extérieures qui peuvent accélérer le processus naturel du vieillissement. Elle est particulièrement sensible aux attaques de radicaux libres générés à la fois par le fonctionnement normal de notre organisme et par des éléments extérieurs, tels que la pollution, le tabac, mais surtout le rayonnement solaire. Ces radicaux libres sont responsables de modifications tissulaires et cellulaires qui conduisent au vieillissement cutané.

Après exposition aux UV, il y a une augmentation de l'expression des métalloprotéinases dans l'épiderme et le derme. Ces enzymes qui altèrent la production de collagène dans le derme contribuent à l'apparition de rides. L'induction de ces enzymes est maintenue par des expositions répétées. Il y a un épaississement du derme dû à l'accumulation de matériel élastique. La production d'interleukines par l'épiderme, suite à une irradiation UV, contribue à la destruction du tissu conjonctif. Il semblerait que les effets observés dans le derme soient plutôt attribuables aux UVA, qui pénètrent plus profondément que les UVB. Chez la souris hairless, comme chez l'homme, on observe l'apparition d'hyperplasie et de rides après irradiation avec des longueurs d'ondes comprises entre 295 et 300 nm, les fibres de collagène sont endommagées et il y a production de fibres élastiques anormales : on parle d'élastose. Après 10 semaines d'irradiation avec des UVA, il n'y a plus de fibres élastiques normales.

Il existe toujours un besoin de disposer de nouveaux actifs ou combinaisons d'actifs efficaces pour lutter contre le vieillissement cutané.

### RESUME DE L'INVENTION

De façon surprenante, les inventeurs ont mis en évidence une synergie pour lutter contre les signes du vieillissement cutané en associant un extrait de vanille de Tahiti au 4-[(1E,3S)-3-éthenyl-3,7-diméthylocta-1,6-dienyl]phénol.

La présente invention a ainsi pour but de proposer l'utilisation par voie topique d'une nouvelle association d'actifs pour lutter contre le vieillissement de la peau.

La présente invention a donc pour objet une association comprenant du 4-[(1E,3S)-3-éthenyl-3,7-diméthylocta-1,6-dienyl]phénol et un extrait de vanille de Tahiti, plus particulièrement un extrait de gousse de vanille de Tahiti,.

La présente invention a également pour objet une composition cosmétique ou dermatologique comprenant, à titre de principe actif, une association selon l'invention, et au moins un véhicule cosmétiquement ou dermatologiquement acceptable.

La présente invention a également pour objet une association selon l'invention ou une composition selon l'invention, pour son utilisation topique pour lutter contre le vieillissement cutané, et/ou prévenir et/ou diminuer les rides, et/ou raffermir la peau, et/ou relancer l'activité cellulaire épidermique et dermique.

La présente invention a également pour objet l'utilisation cosmétique d'une association selon l'invention ou d'une composition selon l'invention, pour lutter contre le vieillissement cutané, et/ou prévenir et/ou diminuer les rides, et/ou raffermir la peau, et/ou relancer l'activité cellulaire épidermique et dermique.

### DEFINITIONS

Les termes « 4-[(1E,3S)-3-éthenyl-3,7-diméthylocta-1,6-dienyl]phénol » et « bakuchiol » sont utilisés de manière interchangeable dans la présente description.

Par « extrait de vanille de Tahiti », on entend désigner le produit d'extraction de gousses mûres de *Vanilla tahitensis.*

Par « produit d'extraction », on entend le produit obtenu après extraction de gousses mûres de *Vanilla tahitensis* avec un solvant, appelé solvant d'extraction, (c'est-à-dire une solution liquide dans le solvant d'extraction) éventuellement sous une forme concentrée ou sèche après évaporation partielle ou totale du solvant d'extraction.

Par « extrait hydrophile », on entend un extrait obtenu par l'utilisation d'au moins un solvant hydrophile comme solvant d'extraction. De préférence, il s'agit d'un extrait obtenu par extraction à l'aide d'un ou plusieurs solvants hydrophiles.

Par « extrait aqueux », on entend un extrait obtenu par l'utilisation d'au moins un solvant aqueux comme solvant d'extraction. De préférence, il s'agit d'un extrait obtenu par extraction à l'aide d'un ou plusieurs solvants aqueux.

Par « extrait hydro-glycolique », on entend un extrait obtenu par l'utilisation d'au moins un solvant hydro-glycolique comme solvant d'extraction. De préférence, il s'agit d'un extrait obtenu par extraction à l'aide d'un ou plusieurs solvants hydro-glycoliques.

Par « solvant hydrophile », on entend un solvant choisi dans le groupe constitué de l'eau, des alcools en C1 à C5 (par exemple éthanol), des glycols en C3 à C5 (par exemple propylène glycol, butylène glycol, pentylène glycol, propane-1,3-diol), du glycérol, de l'acétone, des esters d'alkyles en C1 à C5 (par exemple acétate d'éthyle, acétate d'isopropyle) et des mélanges de ceux-ci.

Par « solvant aqueux », on entend un solvant choisi dans le groupe constitué de l'eau seule et de l'association de l'eau avec un ou plusieurs alcools en C1 à C5 (par exemple éthanol), un ou plusieurs glycols en C3 à C5 ou un mélange de ceux-ci.

Par « solvant hydro-glycolique », on entend un mélange d'eau et d'un ou plusieurs glycols en C3 à C5, notamment le propylène glycol, le butylène glycol, le pentylène glycol, le propane-1,3-diol ou une combinaison de ceux-ci.

Par « extrait sec », on entend au sens de la présente invention, un extrait dépourvu de solvant d'extraction ou de support ou en contenant uniquement à l'état de trace non significative. Un tel extrait sec contient ainsi uniquement de la matière issue des gousses de vanille de Tahiti. Il peut contenir également des traces non significatives de solvant d'extraction.

Par « application topique », on entend une application sur la peau, les muqueuses et/ou les phanères, de préférence la peau.

Dans la présente invention, on entend désigner par « cosmétiquement ou dermatologiquement acceptable » ce qui est utile dans la préparation d'une composition cosmétique ou dermatologique, qui est généralement sûre, non toxique et ni biologiquement ni autrement non souhaitable et qui est acceptable pour une utilisation cosmétique ou dermatologique, notamment par application topique.

On entend par « véhicule cosmétiquement ou dermatologiquement acceptable », tout adjuvant ou excipient permettant la fabrication, la conservation et/ou l'administration d'une composition cosmétique ou dermatologique.

### DESCRIPTION DETAILLEE

Selon un premier aspect, l'invention concerne une association d'un extrait de vanille de Tahiti et du 4-[(1E,3S)-3-éthenyl-3,7-diméthylocta-1,6-dienyl]phénol, notamment pour son utilisation pour lutter contre le vieillissement cutané.

Le vanillier de Tahiti, *Vanilla tahitensis,* appartient à la famille des Orchidacées. L'une des particularités botaniques du genre Vanilla est qu'elle est la seule orchidée à fructifier. La vanille de Tahiti est une espèce de vanille parmi les 119 espèces recensées à ce jour.

*Vanilla tahitensis* est une liane vivace grimpante de couleur verte, cylindrique, de diamètre variant entre 1 à 1,5 cm, qui atteint en général une hauteur de 15 à 20 mètres. Tout comme le lierre, *Vanilla tahitensis* nécessite un tuteur pour se développer : c'est donc une espèce épiphyte (poussant sur une autre plante sans la parasiter). Les tiges noueuses et charnues s'accrochent au support par des racines adventives. Ces dernières se développent au niveau de noeuds et leur rôle d'accroche leur ont valu leur surnom de « crampons ». Ces racines aériennes ont en fait un double rôle : elles fixent la liane sur le tuteur et elles absorbent l'humidité de l'air grâce à leur structure spéciale. Les feuilles vertes sont allongées et persistantes. Longues de 15 à 20 cm et larges de 5 à 8 cm, elles prennent naissance au niveau des noeuds. A l'aisselle de chaque feuille se trouve un oeil qui peut donner naissance à un rameau secondaire. Les fleurs sont grandes : leur diamètre peut parfois atteindre 10 cm. De couleur jaune clair, elles sont réunies en grappes ou en épis auxiliaires regroupant parfois une trentaine de fleurs. Chaque fleur est composée de 3 sépales pétaloïdes et de 3 pétales dont un est plus développé que les deux autres. Ses bords sont soudés pour former un cornet nommé le « labelle ». Celui-ci entoure complètement les organes sexuels composés d'une étamine constituée d'un filet portant une pollinie et d'un style surmonté de deux stigmates fertiles. Une languette assez épaisse sépare la pollinie et les stigmates : c'est le « rostellum » qui empêche toute fécondation naturelle. Pour fructifier, la vanille a donc besoin d'un agent extérieur. Une fois fécondée, l'inflorescence porte un certain nombre de fruits : c'est le « balai ». Le fruit est constitué d'une capsule, la gousse, renfermant 3 loges et un grand nombre de graines noirâtres. Les fruits verts ont une longueur variant de 10 à 20 cm et une largeur s'étalant de 1 à 1,5 cm. La gousse est fixée sur le « balai » par un « crochet ». L'une des particularités de la vanille tahitienne par rapport à ses congénères se situe au niveau de son mûrissement. Alors que les autres espèces de vanille doivent être absolument récoltées avant maturité, au risque sinon de voir leurs gousses éclater, *Vanilla tahitensis* est récoltée sans dommage à maturité, sur pied.

Plusieurs éléments sont prérequis à la culture de *Vanilla tahitensis.* Cette dernière a en effet des exigences climatiques très marquées : le sol doit être léger, drainable et riche en humus et en matières minérales. Des pluies abondantes sont indispensables. Les vanilliers doivent être cultivés à une altitude inférieure à 600 mètres, sous une lumière douce souvent obtenue par ombrage. Enfin la température idéale est comprise entre 20 et 30°C. Le vanillier se bouture facilement, en quelques semaines il a développé ses racines. Au bout de 3 ans a lieu la première floraison.

Le fruit se développe pour atteindre 15 à 20 cm en quelques mois. Lorsqu'il atteint sa taille définitive, le fruit prend un vert brillant identique à celui des tiges et des feuilles. Il ne dégage alors aucune odeur. Au bout de 8 à 9 mois, le fruit devient vert pale, puis vire au jaune et enfin au marron à son extrémité, c'est le signal de la cueillette. Celle-ci se fait à la main, par une légère torsion. Une fois récoltée, les gousses sont mises à l'obscurité pendant plusieurs jours et deviennent uniformément brunes. Ensuite, les gousses sont mises à sécher 2 heures par jour au soleil. Deux mois au moins sont nécessaires pour obtenir les gousses noires à l'aspect flétri. C'est durant cette période de séchage que la vanille acquiert son arôme reconnaissable entre tous. La vanille de Tahiti a un arôme particulier lié à sa phytochimie : l'héliotropine, molécule très odorante et capiteuse, ne se retrouve en effet que dans *Vanilla tahitensis.*

Selon la bibliographie, *Vanilla tahitensis* renferme principalement :
- des polyphénols et dérivés polyphénoliques : l'héliotropine, la vanilline, l'alcool anisique, l'acide anisique, l'acide parahydroxybenzoïque, etc. ;
- des sucres ;
- des matières grasses ;
- des minéraux.

Selon l'invention, l'extrait de vanille de Tahiti est un extrait hydro-glycolique. Cet extrait est obtenu avec au moins un solvant hydro-glycolique.

Selon un mode de réalisation particulier, cet extrait est obtenu par extraction des gousses de vanille de Tahiti, à l'aide d'un ou plusieurs solvant(s) hydro-glycolique(s), tel qu'un mélange eau / propylène glycol, par exemple dans un rapport eau / propylène glycol de 25/75 à 40/60.

L'extrait obtenu peut être utilisé en l'état ou sous forme d'extrait concentré ou d'extrait sec après évaporation partielle ou totale du solvant d'extraction.

Les extraits de vanille de Tahiti utilisés dans les compositions selon l'invention comprendront avantageusement une teneur d'extrait sec de 0,5% à 5%, de préférence de 0,6 à 2% en poids par rapport au poids total de l'extrait.

Le 4-[(1E,3S)-3-éthenyl-3,7-diméthylocta-1,6-dienyl]phénol ou Bakuchiol est un composé méroterpène hautement purifié, décrit dans le brevet US 8529967, connu sous le numéro CAS : 10309-37-2. Les méroterpènes sont généralement obtenus à partir de plantes ou d'extraits de plantes. Ils peuvent également être obtenus à partir de champignons ou encore être synthétisés. Des plantes servant de sources pour les méroterpènes sont par exemple *Psoralea coryfolia, Psoralea grandulosa* et *Otholobium pubescens.*

En pratique, le méroterpène peut être apporté par l'ajout de méroterpène isolé et purifié, de préférence ayant un degré de pureté au moins égal à 60%, voire au moins 70%, 80%, 90% voire au moins égal à 95%, ou par l'ajout d'un extrait végétal comprenant le méroterpène.

Avantageusement, le bakuchiol est apporté par l'ajout d'un extrait de plante le comprenant, de préférence un extrait de graines de *Psoralea coryfolia.*

Selon un autre mode de réalisation, le bakuchiol est isolé et purifié à partir de la plante *Psoralea coryfolia,* avantageusement de ses graines, comme par exemple le produit titré en bakuchiol, commercialisé par la société SYTHEON LTD sous la dénomination Sytenol^{®}. Dans ce produit le bakuchiol a une pureté supérieure ou égale à 95%.

La présente invention vise ainsi une association comprenant un extrait hydro-glycolique de vanille de Tahiti et le bakuchiol.

Selon un autre mode de réalisation particulier de l'invention, cette dernière vise une association selon l'invention selon l'un quelconque des modes de réalisations décrit précédemment pour son utilisation destinée à lutter contre les signes du vieillissement cutané, plus particulièrement pour lutter contre le vieillissement cutané photo-induit. De préférence cette association est utilisée par voie topique.

Selon un autre aspect, l'invention vise une utilisation cosmétique de l'association selon l'invention selon l'un quelconque des modes de réalisations décrit précédemment pour limiter le vieillissement cutané, et/ou prévenir et/ou diminuer les rides, et/ou raffermir la peau, et/ou relancer l'activité cellulaire épidermique et dermique.

Selon un autre aspect, l'invention vise une utilisation de l'association selon l'invention selon l'un quelconque des modes de réalisations décrit précédemment pour la préparation d'une composition cosmétique ou dermatologique, de préférence topique, destinée à lutter contre le vieillissement cutané, photo-induit ou non, et plus particulièrement destinée à limiter le vieillissement cutané, et/ou prévenir et/ou diminuer les rides, et/ou raffermir la peau, et/ou relancer l'activité cellulaire épidermique et dermique.

Selon un autre aspect, l'invention vise une méthode cosmétique pour lutter contre le vieillissement cutané, photo-induit ou non, et plus particulièrement destinée pour limiter le vieillissement cutané, et/ou prévenir et/ou diminuer les rides, et/ou raffermir la peau, et/ou relancer l'activité cellulaire épidermique et dermique, comprenant l'administration, de préférence par application topique, en particulier sur la peau, à une personne en ayant besoin d'une association selon l'invention selon l'un quelconque des modes de réalisations décrit précédemment.

La présente invention concerne aussi une composition cosmétique ou dermatologique comprenant à titre de principes actifs un extrait de vanille de Tahiti et du bakuchiol, et au moins un véhicule cosmétiquement ou dermatologiquement acceptable.

Selon un mode de réalisation préféré, le véhicule cosmétiquement ou dermatologiquement acceptable se présente sous une forme destinée à une application topique. L'objet de l'invention est tout particulièrement destiné à une application topique sur des zones cutanées considérées comme fragiles, comme par exemple le visage, le cou, le contour des yeux et des lèvres. Tout véhicule acceptable du point de vue cosmétique ou dermatologique, choisi par exemple parmi les adjuvants et excipients couramment utilisés en galénique, peut être utilisé dans la composition selon l'invention.

Les compositions cosmétiques ou dermatologiques, selon l'invention pourront se présenter sous les formes qui sont habituellement connues pour une administration topique, c'est-à-dire notamment les lotions, les mousses, les gels, les dispersions, les émulsions, les sprays, les sérums, les masques, les crèmes, ou les gelées, notamment les gelées micellaires, avec des excipients permettant notamment une meilleure biodisponibilité afin d'améliorer les propriétés et l'accessibilité des principes actifs. Avantageusement, il s'agira d'une crème, d'un baume, ou d'un sérum.

Ces compositions contiennent généralement, outre les composés de l'association selon la présente invention, un milieu physiologiquement acceptable, en général à base d'eau ou de solvants, par exemple des alcools, des éthers ou des glycols. Elles peuvent également contenir des agents tensioactifs, des agents complexants, des conservateurs, des agents stabilisants, des émulsifiants, des épaississants, des gélifiants, des humectants, des émollients, des oligo-éléments, des huiles essentielles, des parfums, des colorants, des agents matifiants, des filtres chimiques ou minéraux, des agents hydratants, des eaux thermales, etc.

Avantageusement, les compositions selon la présente invention comprendront 0,01% à 10% en poids, de préférence 0,1° à 5% en poids, de manière préférée 0,2% à 2% en poids, de manière encore préférée 0,3% à 1,5% en poids d'extrait de Vanilla tahitensis, par rapport au poids total de la composition. D'une manière préférée, la composition comprendra environ 0,5% en poids d'extrait de Vanilla tahitensis, par rapport au poids total de la composition. D'une manière tout aussi préférée, la composition comprendra environ 1% en poids d'extrait de Vanilla tahitensis, par rapport au poids total de la composition.

Selon un autre mode de réalisation, les compositions selon la présente invention comprendront 0,00005 à 0,5%, de préférence 0,0005 à 0,25%, de manière encore préférée 0,001 à 0,1%, de manière encore plus préférée 0,0015 à 0,075% d'extrait de vanille de Tahiti en poids d'extrait sec par rapport au poids total de la composition.

Avantageusement, les compositions selon la présente invention comprendront 0,01% à 10% en poids, de préférence 0,1° à 5% en poids, de manière préférée 0,2% à 3% en poids, de manière encore préférée 0,5% à 2% en poids de bakuchiol, par rapport au poids total de la composition. D'une manière préférée, la composition comprendra environ 1% en poids de bakuchiol, par rapport au poids total de la composition. D'une manière toute aussi préférée, la composition comprendra environ 1,5% de bakuchiol, par rapport au poids total de la composition.

De manière particulière, l'invention concerne une composition dans laquelle le ratio massique extrait de vanille de Tahiti : bakuchiol est compris entre 1 : 15 et 10 : 1, préférentiellement entre 1 : 10 et 5 : 1.

Les compositions selon l'invention peuvent être utilisées en cosmétologie et en dermatologie pour prévenir ou améliorer les peaux ridées et permettent de maintenir la fermeté et l'humidité de la peau.

Les compositions selon l'invention peuvent être utilisées dans des préparations topiques, dans le domaine de la dermatologie ou de la cosmétologie, dans le but de prévenir et/ou de diminuer les rides, de lutter contre le vieillissement cutané photo-induit ou non, de raffermir la peau, de relancer l'activité cellulaire épidermique et dermique.

L'invention concerne également l'utilisation cosmétique de la composition selon l'invention pour lutter contre le vieillissement de la peau, et plus particulièrement contre le vieillissement cutané photo-induit.

L'invention concerne également un procédé cosmétique pour lutter contre le vieillissement cutané photo-induit ou non, comprenant l'application sur la peau de la composition selon l'invention.

De telles compositions peuvent être fabriquées selon des procédés bien connus de l'homme du métier.

Les exemples suivants illustrent l'invention sans en limiter la portée.

### Exemple 1 : Effet antivieillissement d'un extrait de vanille, de bakuchiol et de leur association

Le vieillissement des tissus organiques, et en particulier celui de la peau est un processus biologique naturel et complexe. Il se compose de deux types de vieillissement : le vieillissement chronologique ou intrinsèque qui est un processus inévitable lié principalement à l'âge, et le vieillissement extrinsèque lié à des facteurs environnementaux tels que les UV responsables d'un vieillissement prématuré de la peau appelé photo-vieillissement. Une des causes qui explique en partie le déclin fonctionnel de la peau avec l'âge est que la capacité de régénération des cellules, essentielle pour les fonctions tissulaires, est altérée au cours du temps. Toutefois, il a récemment été suggéré que le vieillissement est aussi associé à l'accumulation de cellules dites sénescentes qui présentent un phénotype spécifique : elles sécrètent des cytokines et autres médiateurs qui influencent le fonctionnement de la peau (sécretome) et sont incapables de se diviser dans le tissu (arrêt du cycle cellulaire). Le cycle cellulaire est un mécanisme complexe qui est donc fortement influencé par le vieillissement. Parmi les marqueurs moléculaires du processus de sénescence, la protéine P16 est un inhibiteur spécifique du cycle cellulaire qui entraîne un blocage de la prolifération cellulaire en phase G1 et participe ainsi à l'induction du programme de sénescence responsable du vieillissement des cellules.

Le but de cette étude est d'évaluer les effets du bakuchiol, d'un extrait de vanille de Tahiti et de leur association sur la prévention du vieillissement après un stress aigu aux UVA sur des fibroblastes normaux humains.

Le modèle biologique utilisé pour cette étude consiste en des fibroblastes de derme humain normaux obtenus à partir de donneurs sains.

Les fibroblastes sont ensemencés dans un milieu de culture standard pendant 36 heures, les produits à tester (véhicule (DMSO), bakuchiol (0,5 µg/ml), extrait de vanille (0,05%, p/v) et leur association (mêmes concentrations)) sont ajoutés au milieu de culture une heure avant le test de radiation aux UVA. Ce test consiste en un stress de photo-vieillissement : les fibroblastes sont irradiés par des UVA à une dose de 60J/cm² (Waldmann UVA lamp). Une condition sans irradiation est également réalisée. Les fibroblastes sont ensuite prélevés 24h après l'irradiation pour des analyses d'immunofluorescence. Toutes les conditions expérimentales sont réalisées avec N=3 donneurs. La quantification de fluorescence a été réalisée à l'aide du logiciel Nikon NIS Element afin d'obtenir une moyenne de fluorescence par cellule et par condition.

L'extrait de vanille de Tahiti testé dans cette étude, a été obtenu à partir de l'extraction hydro-glycolique de gousses de vanille de Tahiti, à l'aide d'un mélange eau / propylène glycol comme solvant d'extraction. Cet extrait comprend environ 64,7° de propylène glycol, 34,5% d'eau et environ 0,8% d'extrait sec. Il s'agit d'une matière commerciale Vanirea^{®} UP vendue par la société Solabia. Le nom INCI de cette matière est : propylène glycol / aqua / vanilla tahitensis. Le numéro CAS de vanilla tahitensis est : 94167-14-3.

Le bakuchiol testé dans cette étude correspond à la matière commerciale Sytenol^{®} UP du fournisseur Sytheon. Son numéro CAS est le 10309-37-2.

Le traitement des fibroblastes humains avec un stress aigu par UVA induit une augmentation significative de l'expression de P16, passant de 500 UA à presque 1000 UA, P<0,001, versus la condition non stimulée. Ce résultat permet de valider le test. Les statistiques sont réalisées en utilisant une analyse de variance à un facteur (one-way ANOVA) suivie par un test de Dunnett.

La protéine P16 est normalement faiblement exprimée dans les cellules et son expression est augmentée lors d'un stress ou du vieillissement pour induire un arrêt du cycle cellulaire et favoriser l'activation du programme de sénescence dans les cellules. D'ailleurs la recherche de l'expression de la protéine P16 par immunohistochimie ou immunocytochimie est un moyen pour établir le diagnostic de la présence de cellules sénescentes dans les tissus.

Le traitement des fibroblastes avec l'extrait de vanille testé à 0,05% tend à diminuer l'expression de P16 fortement induite par le stress aux UVA ; cette diminution n'est que de 29,2%, P=NS versus le groupe non traité. De même le traitement des fibroblastes par le bakuchiol testé à 0,5 µg/mL réduit l'expression de P16 suite à l'irradiation, mais cette réduction n'atteint pas la significativité, -44,4%, P=NS versus le groupe non traité. En revanche, le traitement des fibroblastes par l'association de ces 2 composés, aux mêmes concentrations, permet une prévention significative de l'augmentation de l'expression de P16 induite par un stress aux UVA, cette réduction atteignant 95,2%, P<0,001 versus le groupe non traité. Les composés seuls ne peuvent pas prévenir l'expression de P16 d'une manière significative. Cependant l'addition de leurs propres effets (29,2 + 44,4 = 73,6% de protection) est très inférieure à la protection engendrée par leur association (95,2 » 73,6% de protection). Les inventeurs mettent ainsi en évidence une synergie d'action en associant un extrait de vanille et de bakuchiol.

Ces résultats permettent de démontrer que l'association de bakuchiol et d'un extrait de vanille prévient la sénescence des cellules liées aux UVA. En agissant sur la protéine P16, cette association va diminuer l'activation du programme de sénescence dans les cellules et donc permettre de relancer l'activité des cellules épidermiques et dermiques. Les inventeurs mettent ainsi en évidence que cette association est donc dotée de propriétés antivieillissement.

### Exemple 2 : Effet antiinflammatoire d'un extrait de vanille, de bakuchiol et de leur association

Le vieillissement est fortement relié à une altération du système immunitaire et de l'inflammation. Par exemple, le derme vieilli abrite plus de mastocytes et de neutrophiles. Ce phénomène est corrélé avec une up-régulation de l'interleukine 8 (IL8) dans la peau âgée. L'IL8 est une cytokine pro-inflammatoire et chimiotactique. Bien que la source principale d'IL8 soit le monocyte, elle est aussi produite par les macrophages, les cellules endothéliales, les kératinocytes et les fibroblastes. En raison de leur localisation, la production d'IL8 par les fibroblastes peut jouer un rôle important dans la communication avec les cellules endothéliales du derme, augmentant la migration des neutrophiles dans le derme et accentuant l'inflammation. De plus la synthèse d'IL8 par les fibroblastes du derme est considérablement influencée par l'irradiation des UV.

Le but de cette étude est d'évaluer les effets du bakuchiol, d'un extrait de vanille et de leur association sur la prévention de l'inflammation, notamment sur la production d'IL8 après un stress aigu aux UVA sur des fibroblastes normaux humains.

Le protocole utilisé est rigoureusement le même que celui décrit dans l'exemple 1. Les produits testés sont les mêmes, les concentrations également. L'expression d'IL8 est alors quantifiée par immunofluorescence.

Le traitement des fibroblastes humains avec un stress aigu par UVA induit une augmentation significative de l'expression d'IL8, passant de 100% à 5300% (P<0,05) par rapport à la condition sans irradiation. De plus, il apparait que le traitement par les UVA induit des changements morphologiques importants des fibroblastes, passant d'un modèle en forme d'étoile à un modèle fusiforme. Ces résultats permettent de bien valider ce modèle.

Les traitements des fibroblastes avec l'extrait de vanille ou le bakuchiol ou leur association, résultent en une prévention des changements morphologiques induits par l'exposition aux UVA, ce qui indique une activité potentielle de ces composés.

Le traitement des fibroblastes humains avec l'extrait de vanille, testé à 0,05% (p/v) résulte en une tendance évidente à prévenir l'expression d'IL8 induite par le stress à l'irradiation, avec une inhibition de 83,8%, (P=NS versus conditions non traitées). Avec le bakuchiol (0,5µg/ml), l'expression d'IL8 suite à l'exposition aux UVA, est significativement diminuée (88,3%, P<0,05 versus conditions non traitées). Le traitement des fibroblastes par l'association de ces 2 composés aux mêmes concentrations, permet une prévention significative de l'augmentation de l'expression d'IL8 induite par un stress aux UVA, cette réduction atteint 95,1%, P<0,05 versus le groupe non traité.

Ainsi, les inventeurs mettent en évidence que l'association d'un extrait de vanille et de bakuchiol induit un effet antiinflammatoire plus important que celui des deux composés pris isolément.

### Exemple 3 : Exemples de compositions comprenant du bakuchiol et un extrait de Vanille de Tahiti selon l'invention

### Composition pour une crème jour :

| INCI désignation | Pourcentage en poids |
|---|---|
| Eau | QSP 100% |
| Glycerin | 5,0 |
| Disodium EDTA | 0,1 |
| Hexanediol | 0,5 |
| Polyacrylate | 1,2 |
| Behenic Alcohol | 1,0 |
| Caprylic/Capric Triglycérides | 6,0 |
| Dicaprylyl Carbonate | 7,0 |
| Capryl Glycol | 0,5 |
| Beeswax | 1,0 |
| Tribehenin PEG-20 Esters | 5,0 |
| Sytenol | 1,0 |
| Vanilla extract | 0,5 |
| Fragance | 0,2 |

### Composition pour une crème nuit :

| INCI désignation | Pourcentage en poids |
|---|---|
| Eau | QSP 100% |
| Glycerin | 10,0 |
| Disodium EDTA | 0,1 |
| Hexanediol | 0,5 |
| Polyacrylate | 1,2 |
| Behenic Alccol | 2,0 |
| Caprylic/Capric Triglycérides | 10,0 |
| Dicaprylyl Carbonate | 7,0 |
| Capryl Glycol | 0,5 |
| Beeswax | 1,0 |
| Ceterayl Glucoside & Cetearyl Alcohol | 2,0 |
| Glyceryl Stéarate & PEG-100 Stéarate | 3,0 |
| Sytenol | 1,0 |
| Vanilla extract | 0,5 |
| Fragance | 0,2 |

### Composition pour un sérum :

| INCI désignation | Pourcentage en poids |
|---|---|
| Eau | QSP 100% |
| Glycerin | 10,0 |
| Disodium EDTA | 0,1 |
| Hexanediol | 0,5 |
| Polyacrylate | 1,2 |
| Cetyl Alcohol | 1,5 |
| Caprylic/Capric Triglycérides | 10,0 |
| Dicaprylyl Carbonate | 3,0 |
| Capryl Glycol | 0,5 |
| Sytenol | 1,5 |
| Vanilla extract | 1,0 |
| Fragance | 0,2 |

## Revendications

1. Association comprenant du 4-[(1E,3S)-3-éthenyl-3,7-diméthylocta-1,6-dienyl]phénol et un extrait hydro-glycolique de gousse de vanille de Tahiti obtenu par extraction avec au moins un solvant hydro-glycolique, un solvant hydro-glycolique étant un mélange d'eau et d'un ou plusieurs glycols en C3 à C5.

2. Association selon la revendication 1, **caractérisée en ce que** le glycol en C3 à C5 est le propylène glycol, le butylène glycol, le pentylène glycol, le propane-1,3-diol ou une combinaison de ceux-ci.

3. Association selon l'une quelconque des revendications 1 et 2, pour son utilisation topique pour lutter contre le vieillissement cutané.

4. Association selon la revendication 3, pour son utilisation topique pour lutter contre le vieillissement cutané photo-induit.

5. Utilisation cosmétique non- thérapeutique de l'association selon l'une quelconque des revendications 1 et 2, pour limiter le vieillissement cutané, et/ou prévenir et/ou diminuer les rides, et/ou raffermir la peau, et/ou relancer l'activité cellulaire épidermique et dermique.

6. Composition cosmétique ou dermatologique comprenant, à titre de principe actif, une association selon l'une quelconque des revendications 1 à 2, avec au moins un véhicule cosmétiquement ou dermatologiquement acceptable.

7. Composition selon la revendication 6, **caractérisée en ce qu'**elle comprend 0,01% à 10%, notamment 0,1% à 5%, de préférence 0,2% à 2%, en poids d'un extrait de gousse de vanille de Tahiti par rapport au poids total de la composition.

8. Composition selon l'une quelconque des revendications 6 et 7, **caractérisée en ce qu'**elle comprend 0,01% à 10%, notamment 0,1% à 5%, de préférence 0,2% à 3%, en poids de 4-[(1E,3S)-3-ethenyl-3,7-dimethylocta-1,6-dienyl]phenol par rapport au poids total de la composition.

9. Composition selon l'une quelconque des revendications 6 à 8, **caractérisée en ce qu'**elle se présente sous une forme propre à une application topique.

10. Composition selon l'une quelconque des revendications 6 à 9, pour son utilisation pour lutter contre le vieillissement cutané.

11. Composition selon la revendication 10, pour son utilisation pour lutter contre le vieillissement cutané photo-induit.

12. Utilisation cosmétique non-thérapeutique d'une composition selon l'une quelconque des revendications 6 à 9 pour limiter le vieillissement cutané, et/ou prévenir et/ou diminuer les rides, et/ou raffermir la peau, et/ou relancer l'activité cellulaire épidermique et dermique.

## Patentansprüche

1. Vereinigung, die 4-[(1E,3S)-3-ethenyl-3,7-dimethylocta-1,6-dienyl]phenol und einen Hydroglucolextrakt von Tahitivanilleschote umfasst, erhalten durch Extraktion mit mindestens einem Hydroglycollösungsmittel, wobei ein Hydroglycollösungsmittel ein Gemisch aus Wasser und aus einem oder mehreren C3-C5-Glycolen ist.

2. Vereinigung nach Anspruch 1, **dadurch gekennzeichnet, dass** das C3-C5-Glycol Propylenglycol, Butylenglycol, Pentylenglycol, Propan-1,3-diol oder eine Kombination derselben ist.

3. Vereinigung nach einem der Ansprüche 1 und 2, für ihre topische Verwendung zur Bekämpfung von Hautalterung.

4. Vereinigung nach Anspruch 3, für ihre topische Verwendung zur Bekämpfung der lichtinduzierten Hautalterung.

5. Nichttherapeutische kosmetische Verwendung der Vereinigung nach einem der Ansprüche 1 und 2 zur Begrenzung der Hautalterung und/oder Vorbeugung und/oder Verringerung der Falten und/oder Straffung der Haut und/oder Ankurbelung der Zellaktivität der Epidermis und Dermis.

6. Kosmetische oder dermatologische Zusammensetzung, die als Wirkstoff eine Vereinigung nach einem der Ansprüche 1 bis 2, mit mindestens einem kosmetisch oder dermatologisch akzeptablen Träger umfasst.

7. Zusammensetzung nach Anspruch 6, **dadurch gekennzeichnet, dass** sie 0,01 bis 10 Gew.-%%, insbesondere 0,1 bis 5 Gew.-%, vorzugsweise 0,2 bis 2 Gew.-% eines Tahitivanilleschotenextrakts im Verhältnis zum Gesamtgewicht der Zusammensetzung umfasst.

8. Zusammensetzung nach einem der Ansprüche 6 und 7, **dadurch gekennzeichnet, dass** sie 0,01 bis 10 Gew.-%, insbesondere 0,1 bis 5 Gew.-%, vorzugsweise 0,2 bis 3 Gew.-% 4-[(1E,3S)-3-ethenyl-3,7-dimethylocta-1,6-dienyl]phenol im Verhältnis zum Gesamtgewicht der Zusammensetzung umfasst.

9. Zusammensetzung nach einem der Ansprüche 6 bis 8, **dadurch gekennzeichnet, dass** sie in einer Form vorliegt, die für eine topische Anwendung geeignet ist.

10. Zusammensetzung nach einem der Ansprüche 6 bis 9 für ihre Verwendung zur Bekämpfung der Hautalterung.

11. Zusammensetzung nach Anspruch 10 für ihre Verwendung zur Bekämpfung der lichtinduzierten Hautalterung.

12. Nichttherapeutische kosmetische Verwendung einer Zusammensetzung nach einem der Ansprüche 6 bis 9 zur Begrenzung der Hautalterung und/oder Vorbeugung und/oder Verringerung der Falten und/oder Straffung der Haut und/oder Ankurbelung der Zellaktivität der Epidermis und Dermis.

## Claims

1. Combination including 4-[(1E,3S)-3-ethenyl-3,7-dimethylocta-1,6-dienyl]phenol and a hydro-glycolic Tahiti vanilla pod extract obtained by extraction with at least one hydro-glycolic solvent, a hydro-glycolic solvent being a mixture of water and one or more C3 to C5 glycols.

2. Combination according to claim 1, **characterised in that** the C3 to C5 glycol is propylene glycol, butylene glycol, pentylene glycol, propan-1,3-diol or a combination thereof .

3. Combination according to any of claims 1 and 2, for the topical use thereof for combatting cutaneous ageing.

4. Combination according to claim 3, for the topical use thereof for combatting photo-induced cutaneous ageing.

5. Non-therapeutic cosmetic use of the combination according to any of claims 1 and 2, for limiting cutaneous ageing, and/or preventing and/or reducing wrinkles, and/or toning up the skin, and/or relaunching epidermal and dermal cellular activity.

6. Cosmetic or dermatological composition including, as active ingredient, a combination according to any of claims 1 to 2, with at least one cosmetically or dermatologically acceptable vehicle.

7. Composition according to claim 6, **characterised in that** it includes 0.01% to 10%, notably 0.1% to 5%, preferably 0.2% to 2%, by weight of a Tahiti vanilla pod extract compared to the total weight of the composition.

8. Composition according to any of claims 6 and 7, **characterised in that** it includes 0.01% to 10%, notably 0.1% to 5%, preferably 0.2% to 3%, by weight of 4-[(1E,3S)-3-ethenyl-3,7-dimethylocta-1,6-dienyl]phenol compared to the total weight of the composition.

9. Composition according to any of claims 6 to 8, **characterised in that** it is in a form suitable for topical application.

10. Composition according to any of claims 6 to 9, for the use thereof for combatting cutaneous ageing.

11. Composition according to claim 10, for the use thereof for combatting photo-induced cutaneous ageing.

12. Non-therapeutic cosmetic use of a composition according to any of claims 6 to 9 for limiting cutaneous ageing, and/or preventing and/or reducing wrinkles, and/or toning up the skin, and/or relaunching epidermal and dermal cellular activity.
